# EUROPEAN PATENT APPLICATION

(11) **EP 2 957 182 A1**
(43) Date of publication of application: **23.12.2015**
(21) Application number: 14172920.2
(22) Date of filing: 18.06.2014
(51) Int. Cl.: A23L 1/236, A23L 1/015

(54) **Improved natural sweetener compositions**

(71) Applicant: Technische Hochschule Mittelhessen, 35390 Giessen (DE)
(72) Inventor: Spohner, Sebastian, 60389 Frankfurt (DE); Quitmann, Hendrich, 35418 Buseck (DE); Czermak, Peter, 35576 Wetzlar (DE)
(74) Representative: Roth, Andy Stefan

(57) **Abstract**

The present disclosure relates to novel natural sweetener compositions and methods for producing such sweetener compositions having improved taste quality and their use in various food products and beverages as a sweetness and flavor modifier.

## Description

### FIELD OF THE INVENTION

The present disclosure relates to novel natural sweetener compositions and methods for producing such sweetener compositions having improved taste quality and their use in various food products and beverages as a sweetness and flavor modifier.

### BACKGROUND OF THE DISCLOSURE

Non-caloric sweeteners of natural origin are becoming increasingly popular. The sweet herb *Stevia rebaudiana* Bertoni, produces a number of diterpene glycosides which feature high intensity sweetness and sensory properties superior to those of many other high potency sweeteners.

Steviol is the backbone of all diterpene glycosides isolated from *S. rebaudiana.* They differ in the content of carbohydrate residues, mono-, di-, and trisaccharides containing glucose and/or rhamnose as well as xylose and fructose at positions C13 and C19. Stevioside is the major steviol glycoside (5-10%) other steviol glycosides are Rebaudiosides A (2-4%), Rebaudiosides C (1-2%), and Dulcoside A (0.4-0.7%).

The sweetness of all steviol glycosides is far greater than that of sucrose: Rebaudioside A, 250 times; Rebaudioside B, 300 times; Rebaudioside C (Dulcoside B), 50 times; Rebaudioside D, 250 times; Rebaudioside E, 150 times; Dulcoside A, 50 times; and Steviobioside, 100 times. Further glycosides have been identified in Stevia extract including Rebaudioside B, and F, Steviolbioside and Rubusoside. So far only Stevioside and Rebaudioside A are available in commercial scale. The average sweetness of steviol glycosides is about 250 times greater than the sweetness of sucrose. The leaves have been traditionally used for hundreds of years to sweeten local teas and medicines.

Methods for the extraction and purification of sweet glycosides from the *Stevia rebaudiana* plant using water or organic solvents are described in, for example, U.S. Pat. Nos. 4,361,697; 4,082,858; 4,892,938; 5,972,120; 5,962,678; 7,838,044 and 7,862,845.

However, bitterness in stevia products comprising steviol glycosides (e.g., extracts) has been a longstanding consumer acceptance problem in the industry. Even in a highly purified state, steviol glycosides still possess undesirable taste attributes such as bitterness, sweet aftertaste, licorice flavor, etc. One of the main obstacles for the successful commercialization of stevia sweeteners are these undesirable taste attributes. It was shown that these flavor notes become more prominent as the concentration of steviol glycosides increases (Prakash I., DuBois G. E., Clos J. F., Wilkens K. L., Fosdick L. E. (2008) Development of rebiana, a natural, non-caloric sweetener. Food Chem. Toxicol., 46, S75-S82.)

Some of these undesirable properties can be reduced or eliminated by subjecting steviol glycosides to the reaction of intermolecular transglycosylation, when new carbohydrate residues are attached to initial molecule at C₁₃ and C₁₉ positions. Depending on the number of carbohydrate residues in these positions the quality and potency of the compounds taste will vary.

Pullulanase, isomaltase (Lobov S. V., Jasai R., Ohtani K., Tanaka O. Yamasaki K. (1991) Enzymatic production of sweet Stevioside derivatives: transglycosylation by glucosidases. Agric. Biol. Chem. 55: 2959-2965), .beta.-galactosidase (Kitahata S., Ishikawa S., Miyata T., Tanaka O. (1989) Production of rubusoside derivatives by transglycosylation of various .beta.-galactosidase. Agric. Biol. Chem. 53: 2923-2928), and dextran saccharase (Yamamoto K., Yoshikawa K., Okada S. (1994) Effective production of glucosyl-Stevioside by alpha.-1,6-transglucosylation of dextran dextranase. Biosci. Biotech. Biochem. 58: 1657-1661) have been used as transglycosylating enzymes, together with pullulan, maltose, lactose, and partially hydrolyzed starch, respectively, as donors of glycosidic residues.

US 2013/0030060 A1 described that steviol glycosides can be debittert completely or in part with cyclodextrin glucanotransferase resulting in non-natural steviololigoglycosides.

However, there remains a need in the art for further debittering methods to improve the taste quality of stevia products that are economical, efficient, and utilizes materials approved by governmental agencies for food processing.

The object of the present invention is therefore to provide simple processes of preparation of natural sweetener compositions having improved taste that circumvents the disadvantages of the known processes.

### SUMMARY OF THE DISCLOSURE

The present disclosure relates to natural sweetener compositions and method/processes for manufacturing these natural sweetener compositions with improved taste quality, in particular natural sweetener compositions derived from *Stevia rebaudiana* plant products comprising steviol glycosides.

The present disclosure is aimed to overcome the disadvantages of existing Stevia sweeteners. In particular, the present disclosure describes a process for producing a natural sweetener compositions having improved taste quality from the extract of the *Stevia rebaudiana* Bertoni plant and use thereof in various food products and beverages as a sweetness and flavor modifier.

In one aspect, the present disclosure pertains the use of enzymes with rhamnosyl hydrolase activity for the specific derhamnosylation of Dulcoside A and Dulcoside B in steviol glycoside mixtures to yield a sweeter and less bitter product.

In another aspect, the present disclosure pertains to methods for producing a natural sweetener composition comprising a plurality of sweetener by subjecting a steviol glycoside composition comprising at least the steviol glycoside Dulcoside A and/or Dulcoside B to an enzyme with rhamnosyl hydrolase activity.

The present disclosure, in part, pertains to the production of a natural sweetener composition comprising glucosylated derivatives of steviol glycosides of *Stevia rebaudiana* Bertoni plant. The steviol glycodsides are selected from the group consisting of Stevioside, Rebaudioside A, Rebaudioside B, Rebaudioside C (Dulcoside B), Rebaudioside D, Rebaudioside E, Rebaudioside F, Dulcoside A, Steviolbioside, Rubusoside, as well as other steviol glycosides found in *Stevia rebaudiana* Bertoni plant and mixtures thereof.

In another aspect, the present disclosure pertains to methods for improving the taste quality of a steviol glycoside composition comprising at least the steviol glycoside Dulcoside A and/or Dulcoside B by subjecting the composition to an enzyme with rhamnosyl hydrolase activity.

In a further aspect, the present disclosure relates to natural sweetener compositions obtainable by subjecting a steviol glycoside composition comprising at least the steviol glycoside Dulcoside A and/or Dulcoside B to an enzyme with rhamnosyl hydrolase activity.

In another aspect, the present disclosure pertains to natural sweetener compositions obtainable by a method according to the present disclosure, wherein said composition contains Dulcoside A at a concentration of less than 2 % (wt/vol), preferably less than 0.5 % (wt/vol), more preferably less than 0.1 % (wt/vol) and Dulcoside B at a concentration of less than 2 % (wt/vol), preferably less than 0.5 % (wt/vol), more preferably less than 0.1 % (wt/vol).

### BRIEF DESCRIPTION OF THE DRAWINGS

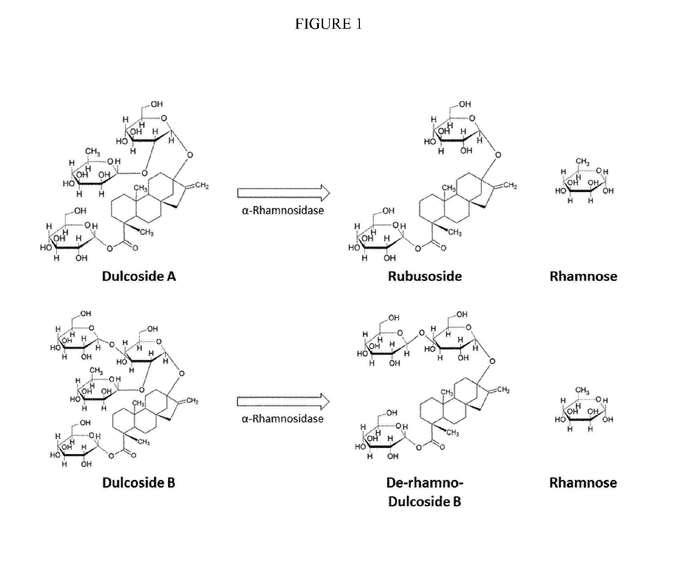

Figure 1 shows the conversion of rhamno steviol glycosides by α-Rhamnosidase. The trimming of Rhamnose from Dulcoside A yields in Rhamnose and Rubusoside. The conversion of Dulcoside B yields Rhamnose and de-rhamno-Dulcoside B, which is a stereoisomer of Stevioside. This stereoisomer can also be produced in the plant *Stevia rebaudisiana.*

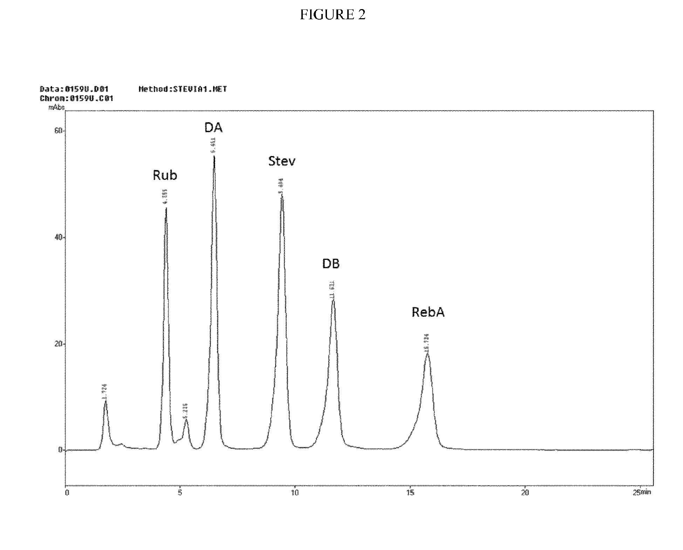

Figure 2 is a chromatogram showing the analysis of a mixture of highly pure steviol glycosides bought from sigma-aldrich with elution times as follows: Rubusoside (Rub) 4.35 min; Dulcoside A (DA) 6.451 min; Stevioside (Stev) 9.404 min; Dulcoside B (DB) 11.631 min; Rebaudioside A (Reb A) 15.734 min.
All components were at a concentration of 0.2 mg/mL.

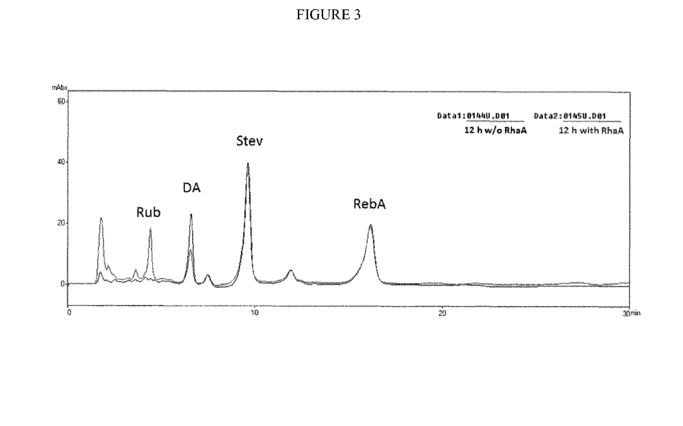

Figure 3 is a chromatogram showing conversion of Dulcoside A (DA) to Rubusoside (Rub) in a steviol glycoside mixture (impag, Switzerland). After 12 hours of reaction the concentration of DA was decreased and Rub the product of de-rhamnosylation was detectable. The first red peak is caused from impureness in the enzyme solution.

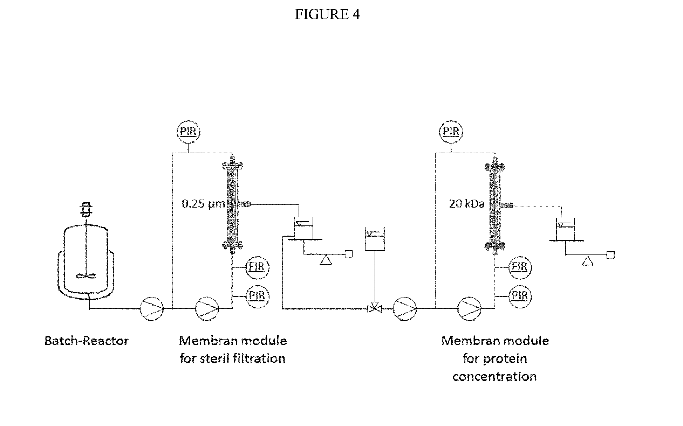

In figure 4 a membrane filtration apparatus is shown, which was used to concentrate and diafiltrate the produced enzyme. The supernatant of a batch bioreactor with recombinant yeast was harvested and sterile filtrated using a ceramic membrane module with a cut-off of 0.25 µm. The flux and the pressure were controlled online and the permeate flow was detected using a balance. In the second step the permeate of the sterile filtration was concentrated in a second ceramic membrane module with a cut-off of 20 kDa. The retentate with the concentrated protein was diafiltrated with 10 volumes of buffer.

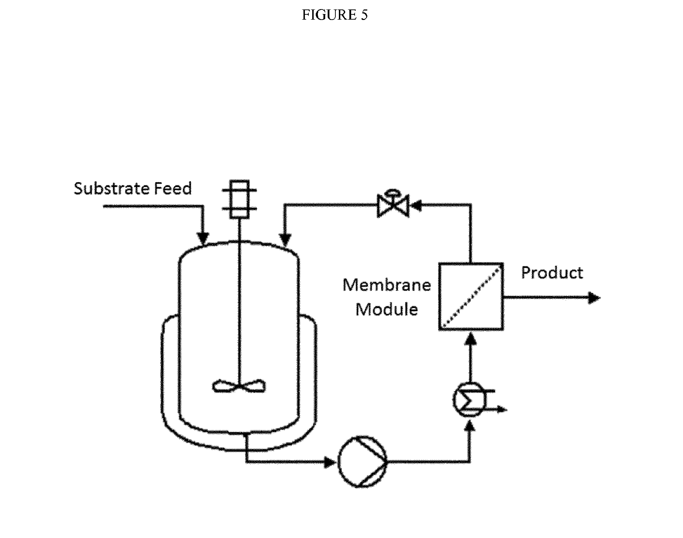

In figure 5 a membrane reactor system for continuous de-rhamnosylation of Dulcosides. The reaction mixture is retained in the stir reactor. Using a membrane module or a cascade of membrane modules substrate and by-products can be separated from the enzyme solution and from each other.

### DETAILED DESCRIPTION OF THE DISCLOSURE

The present disclosure relates to novel natural sweetener compositions and methods for producing such sweetener compositions having improved taste quality and their use in various food products and beverages as a sweetness and flavor modifier.

Advantages of the present disclosure will become more apparent from the detailed description given hereinafter. However, it should be understood that the detailed description and specific examples, while indicating preferred embodiments of the present disclosure, are given by way of illustration only, since various changes and modifications within the spirit and scope of the disclosure will become apparent to those skilled in the art from this detailed description.

As described above, known strategies for taste improvement of steviol glycoside mixtures are separation of bitter tasting steviol glycosides from steviol glycoside mixtures, enzymatic conversion of steviol glycosides to steviololigoglycosides (glucosyl Stevia) and enzymatic conversion of Stevioside to Rubusoside.

A separation of specific steviol glycosides from mixtures gained from extraction of *Stevia rebaudiana* Berton is complex due to amount of compounds and their behaviors in mixtures of steviol glycosides. Several process steps have to be applied, which might result in a very expensive production. Furthermore, environmental issues might cause problems.

The enzymatic conversion of steviol glycosides to steviololigoglycosides (glucosyl Stevia) might be a possible solution for a taste improvement of steviol glycoside mixtures. The problem with this conversion is on the one hand the non-specific glycosylation and on the other hand the procedure itself, which consist of the polymerization and subsequent trimming to the desired residue length. In the first step Stevioside is glycosylated by a gluconotransferase up to ten glucose units. Afterwards the steviooligoglycoside is trimmed with amylase to receive the desired residue length. The composition of the resulting compounds is rather inhomogeneous and is an unnatural product.

The advantage of the methods according to the present disclosure is that the steviol glycosides with a low threshold concentration for bitterness and a high threshold concentration for sweetness (Dulcoside A and Dulcoside B) are enzymatically converted to steviol glycosides with higher threshold concentration for bitterness and/or lower threshold concentration for sweetness. Due to the methods according to the present disclosure the produced sweetener compositions have a lower bitter aftertaste and/or a higher sweetness in comparison to the initial steviol glycoside mixtures.

In 2012 human psychometric and taste receptor responses to steviol glycosides were investigated (Hellfritsch et al, 2012, J. Agric. Food Chem. 60 (27), S. 6782-6793, DOI: 10.1021/jf301297n) indicating that Dulcoside A and Dulcoside B (also Rebaudioside C) are the component with the highest threshold concentration for sweetness and the lowest threshold concentration for bitterness (Table 1).

**Table 1: Relative Sweetness (RS) and Threshold Concentrations (TC) for sweetness and bitterness in human psychometric responses to steviol glycosides**

| | RS | TC sweet (µM) | TC bitter (µM) |
|---|---|---|---|
| Dulcoside A | 1,1 | 32,9 | 49 |
| Dulcoside B | 1,5 | 27,8 | 49 |
| Rubusoside | 1,8 | 27,3 | 61 |
| Stevioside | 2,7 | 11,1 | 112 |
| Rebaudioside A | 3,7 | 8,3 | 194 |
| Rebaudioside D | 4,8 | 5,3 | 162 |

| | | | |
|---|---|---|---|
| Rebaudioside A (4 glucose residues) clearly surpasses Stevioside (3 glucose residues) in taste quality. Steviolbioside and Rubusoside (each 2 glucose residues) are proven to be significantly inferior in taste quality to that of Stevioside. | | | |

The structure of Dulcoside A has an additionally rhamnose residue in comparison to Rubusoside which has a lower threshold concentration of sweetness and a higher threshold concentration of bitter tasting than Dulcosde A. Therefore, the novel method according to the present disclosure for taste improvement comprises the de-rhamnosylation of steviol glycosides using specific enzymes with rhamnosyl hydrolase activity (e.g. α-rhamnosidases). Unwanted side reactions might be prohibited by the usage of enzymes without additional glycosyl hydrolase activity especially against steviol glycosides.

Therefore, in some advantageous embodiments the present disclosure pertains to methods for producing a natural sweetener composition comprising a plurality of sweetener by subjecting a steviol glycoside composition comprising at least the steviol glycoside Dulcoside A and/or Dulcoside B to an enzyme with rhamnosyl hydrolase activity.

The steviol glycoside composition might be a product of an extraction (e.g. commercial available extracts of leaves and whole plants of *Stevia rebaudiana* Berton). Alternatively, it might be a mixture of steviol gylcosides. Other sweetener in the natural sweetener composition may be selected from the group consisting of steviol glycosides including a purified sweet steviol glycoside mixture, Stevioside, Rebaudioside A, Rebaudioside B, Rebaudioside D, Rebaudioside E, Rebaudioside F, Stevia, alpha-glucosyl Stevia, fructosyl Stevia, galactosyl Stevia, beta-glucosyl stevia; Siamenoside; Mogroside IV; Mogroside V; Luo Han Guo sweetener; Monatin and its salts (monatin SS, RR, RS, SR); Glycyrrhizic acid and its salts; Curculin; Thaumatin; Monellin; Mabinlin; Brazzein; Hernandulcin; Phyllodulcin; Glycyphyllin; Phloridzin; Trilobatin; Baiyunoside; Osladin; Polypodoside A; Pterocaryoside A; Pterocaryoside B; Mukurozioside; Phlomisoside I; Periandrin I; Abrusoside A; Cyclocarioside I and combinations thereof. Preferably, the sweeteners have to be dissolved in water or an organic phase.

Steviol glycoside according to the present disclosure may be glucosylated derivatives of steviol glycosides of *Stevia rebaudiana* Bertoni plant. Steviol glycosides may be obtained by extracting leaves of *Stevia rebaudiana* Bertoni with hot water followed by solvent purification of the water-soluble extract. Ion exchange resins may also be used during the purification process. Stevioside and Rebaudioside A are the principal steviol glycoside of the specified material. Dulcoside B (Rebaudioside C) and Dulcoside A are secondary steviol glycosides. Other steviol glycosides may also be present.

According to the present disclosure, the steviol glycodsides may be selected from the group consisting of Stevioside, Rebaudioside A, Rebaudioside B, Rebaudioside C (Dulcoside B), Rebaudioside D, Rebaudioside E, Rebaudioside F, Dulcoside A, steviolbioside, rubusoside, as well as other steviol glycosides found in *Stevia rebaudiana* Bertoni plant and mixtures thereof.

The following are the chemical names for the principal and secondary steviol glycosides:
Stevioside: 13-[(2-O-β-D-glucopyranosyl-β-D-glucopyranosyl) oxy] kaur-16-en-18-oic acid β -D-glucopyranosyl ester
Rebaudioside A: 13-[(2-O-β-D-glucopyranosyl-3-O-β-D-glucopyranosyl-β D-glucopyranosyl)oxy] kaur-16-en-18-oic acid β-D-glucopyranosyl ester
Rebaudioside C (Dulcoside B: 13-[(2-O-α-L-rhamnopyranosyl-3-O-β-D-glucopyranosyl-β-D-glucopyranosyl)oxy] kaur-16-en-18-oic acid β-D-glucopyranosyl ester
Dulcoside A: 13-[2-O-α-L-rhamnopyranosyl-β-D-glucopyranosyl]oxy]kaur-16-en-18-oic acid P-D-glucopyranosyl ester

For example, the percentages of the steviol glycosides in a composition can be determined by high pressure liquid chromatography (Romero et al, 1985, Anal. Biochem. 2, P. 566-571.).

Stevioside and Rebaudioside A for example are low-calorie natural products isolated from *Stevia rebaudiana* Bertoni leaves. They are widely used as sweeteners. Steviol is the backbone of all diterpene glycosides isolated from *S. rebaudiana.* They differ in the content of carbohydrate residues, mono-, di-, and trisaccharides containing glucose and/or rhamnose as well as xylose and fructose at positions C13 and C19. Stevioside is the major steviol glycoside (5-10%) other steviol glycosides are Rebaudiosides A (2-4%), Rebaudiosides C (1-2%), and Dulcoside A (0.4-0.7%). The sweetness of all steviol glycosides is far greater than that of sucrose: Rebaudioside A, 250 times; Rebaudioside B, 300 times; Rebaudioside C, 50 times; Rebaudioside D, 250 times; Rebaudioside E, 150 times; Dulcoside A, 50 times; and steviobioside, 100 times. Further glycosides have been identified in Stevia extract including Rebaudioside B, and F, Steviolbioside and Rubusoside. So far only Stevioside and Rebaudioside A are available in commercial scale. The average sweetness of steviol glycosides is about 250 times greater than the sweetness of sucrose. The leaves have been traditionally used for hundreds of years to sweeten local teas and medicines.

Steviol glycosides possess valuable biological properties. Regular consumption can decrease the content of cholesterol, radionuclides and sugar in blood. It can improve cell regeneration and blood coagulation, strengthen blood vessels and suppress neoplastic growth. Steviol glycosides also exhibit anti-inflammatory, choleretic, and diuretic properties. They also prevent ulceration in the gastrointestinal tract. Among the steviol glycosides, Rebaudioside A and Stevioside have the greatest commercial potential. Rubusoside for example is a sweetener which mainly exists in Chinese sweet tea plant (Rubussuavissimus S. Lee). Rubusoside is also a minor component in extract of Stevia rebaudiana leaf as precursor in Stevioside synthesis. Rubusoside possess certain valuable properties e.g. sweetness-enhancment or usage as a natural solubilizing agent for a number of compounds.

In a further advantageous embodiment, the steviol glycoside composition is a mixture of steviol glycosides comprising at least Stevioside, Rebaudiosides A, Dulcoside B (Rebaudiosides C) and Dulcoside A.

In an advantageous embodiment, the steviol glycoside composition is derived from *Stevia rebaudiana* Bertoni leaves, in particular the steviol glycoside composition is a *Stevia rebaudiana* plant extract.

According to the present disclosure, the steviol glycoside compositions comprising at least the steviol glycoside Dulcoside A and/or Dulcoside B may be subjected to an enzyme with rhamnosyl hydrolase activity, in particular to a α-L-rhamnosidase.

Rhamnosidase, in particular α-L-rhamnosidase (EC 3.2.1.40) cleaves terminal α -L-rhamnose from a large number of natural products, such as flavonoids, saponins, and many other natural glycosides. Rhamnosidases are successfully used to derhamnosilate sterioid glycosides (WO 2011089031 A1). Microbial α-L-rhamnosidases have been mainly purified from commercial enzyme preparations of Penicillium (Young et al. 1989) and Aspergillus strains (Kurosawa et al. 1973; Hsieh and Tsen 1991; Mutter et al. 1994; Manzanares et al. 1997). The production and purification of an Aspergillus terreus α-L-rhamnosidas of potential oenological interest (Gallego et al. 1996 and 2000) and two different Aspergillus aculeatus proteins showing α-L-rhamnosidase activity (Manzanares et al. 2000) have been reported. Recently the biochemical characterization and primary structure of a bacterial α-L-rhamnosidase has been published (Zverlov et al . 2000).

In some advantageous embodiments, the enzyme with rhamnosyl hydrolase activity is an isolated Rhamnosidase, in particular α-L-rhamnosidase. The term "isolated" describes any molecule separated from its natural source. These Rhamnosidase can be produced as technical enzyme using wild-type strains or genetically modified organisms (e.g. with host strain *Pichia pastoris).*

In some advantageous embodiments, the enzyme with rhamnosyl hydrolase activity is derived from bacteria, in particular from bacteria of the order Thermales.

In some other advantageous embodiments, the enzyme with rhamnosyl hydrolase activity is derived from fungi, in particular from a fungi selected from the group consisting of Basidiomycota, Ascomycota and Saccharomycotina.

In a further advantageous embodiment, the enzyme with rhamnosyl hydrolase activity is an artificial technical enzyme.

The term "technical enzyme" means any rhamnosidase, in particular α-L-rhamnosidase enzyme variants obtained by site-directed or random mutagenesis, insertion, deletion, recombination and/or any other protein engineering method, which leads to an enzyme with rhamnosyl hydrolase activity that differ in their amino acid sequence from a wildtype rhamnosidase. The terms "wildtype rhamnosidase" in accordance with the disclosure describe enzyme with rhamnosyl hydrolase activity with an amino acid sequence found in nature or a fragment thereof.

The term "mutation" refers to the substitution or replacement of single or multiple nucleotide triplets, insertions or deletions of one or more codons, homologous or heterologous recombination between different genes, fusion of additional coding sequences at either end of the encoding sequence, or insertion of additional encoding sequences or any combination of these methods, which result in a polynucleic acid sequence encoding the desired protein. Thus, the term "mutations" also refers to all of the changes in the polypeptide sequence encoded by the polynucleic acid sequence modified by one or more of the above-described changes.

In particular, the enzyme with rhamnosyl hydrolase activity (e.g. rhamnosidase, in particular α-L-rhamnosidase) may be a "recombinant" polypeptide, which is defined either by its method of production or its structure. In reference to its method of production, e.g. a product made by a process, the process involved uses recombinant nucleic acid techniques. In reference to structure, recombinant polynucleotides or polypeptides contain sequences from different sources. In particular, it encompasses polypeptides made by generating a sequence comprising two or more fragments, which are not naturally contiguous or operably linked to each other. Thus, for example, products made by transforming cells with any unnaturally occurring vector are encompassed. The term may also be construed to mean fusion protein which has been generated by the synthesis of a DNA molecule encoding the fusion protein and which DNA molecule expresses a fusion protein, or an amino acid sequence specifying the fusion protein, wherein the DNA or amino acid sequence has been obtained using synthetic DNA or amino acid sequence technology which is available and well known in the art.

Furthermore, the encoding nucleotide sequences may be synthesized in vitro without the need for initial template DNA samples e.g. by oligonucleotide synthesis from digital genetic sequences and subsequent annealing of the resultant fragments. Desired protein sequences can be "reverse translated" e.g. using appropriate software tools. Due to the degeneracy of the universal genetic code, synonymous codons within the open-reading frame (i.e. the recombinant protein coding region) can be exchanged in different ways, e.g. to remove cis-acting instability elements (e.g. AUUUA), to remove, introduce or modify the secondary and tertiary mRNA structures (e.g. pseudoknots, stem-loops), to avoid self-complementary regions that might trigger post-transcriptional gene silencing (PGTS), to change the overall AT:GC content, or to adjust the codon-usage to the expression host. Such changes can be designed manually or by using appropriate software tools or through a combination.

To express a protein (enzyme) with rhamnosyl hydrolase activity for the use in the methods according to the present disclosure in a recombinant expression system, a DNA encoding the protein or parts thereof, may be inserted into an expression vector such that the gene is operably linked to transcriptional and translational control sequences. In this context, the term "operably linked" means that a protein gene is ligated into a vector such that transcriptional and translational control sequences within the vector serve their intended function of regulating the transcription and translation of the protein gene. The expression vector and expression control sequences are chosen to be compatible with the expression host cell used. The isolated protein domain sequences are typically inserted into the same expression vector. The protein genes are inserted into the expression vector by standard methods. Additionally, the recombinant expression vector can encode a signal peptide that facilitates co-translational translocation of the nascent polypeptide chain into the endoplasmic reticulum (ER). The folded polypeptide (enzyme with rhamnosyl hydrolase activity) may be secreted from a host cell or may be retained within the host cell. Intracellular retention or targeting can be achieved by the use of an appropriate targeting peptide such as C-terminal KDEL-tag for ER retrieval.

In general, those skilled in the art are well able to construct vectors and design protocols for recombinant gene expression. For further details see, for example, Molecular Cloning: a Laboratory Manual : 2nd edition, Sambrook et al., 1989, Cold Spring Harbor Laboratory Press (or later editions of this work) and Current Protocols in Molecular Biology, Second Edition, Ausubel et al. eds., John Wiley & Sons, 1992, which are incorporated herein by reference.

The phrase "recombinant host cell" (or simply "host cell") includes a cell into which a recombinant expression vector has been introduced. It should be understood that such terms are intended to refer not only to the particular subject cell but also to the progeny of such a cell. Because certain modifications may occur in succeeding generations due to either mutation or environmental influences, such progeny may not, in fact, be identical to the parent cell, but are still included within the scope of the term "host cell" as used herein.

A host cell may also be referred to as a protein-expressing cell. A host cell, according to the present invention, may be, but is not limited to, prokaryotic cells, eukaryotic cells, archaebacteria, bacterial cells, insect cells, yeast, mammal cells, and/or plant cells. Bacteria envisioned as host cells can be either gram-negative or gram-positive, e.g. *Escherichia coli, Erwinia sp., Klebsellia sp., Lactobacillus sp.* or *Bacillus subtilis.* Typical yeast host cells are selected from the group consisting of *Saccharomyces cerevisiae, Hansenula polymorpha* and *Pichia pastoris.*

After expression, proteins may be isolated and purified in accordance with conventional conditions and techniques known in the art. These include methods such as extraction, precipitation, chromatography, affinity chromatography, electrophoresis, and the like.

Figure 4 shows an embodiment of a protein production process.

The enzymes used in the methods according to the present disclosure can be in a form of cell-free culture broth, concentrated liquid cell-free culture broth, spray dried or freeze dried cell-free culture broth, or high purity protein. Free and immobilized enzyme preparations can be used.

Examples for immobilizing of said enzyme is that the enzyme is bound to a carrier, the enzyme is immobilized onto a membrane or the enzyme is dynamically immobilized onto a membrane.

In an advantageous embodiment, the used enzyme with rhamnosyl hydrolase activity has no glycosyl hydrolase activity. In particular, the enyzme has no specific glycosyl hydrolase activity against steviol glycosides. Furthermore, if an enzyme mixture comprising an enzyme with rhamnosyl hydrolase activity is used, no enzymes with specific glycosyl hydrolase activity, or in particular no enyzmes having specific glycosyl hydrolase activity against steviol glycosides are used.

Examples for enzymes having glycosyl hydrolase activity are ß-glucosidase, hesperidinase, naringinase, pectinase and cellulase. For example, Naringinases, Hesperidinases and Rhamnosidases are complexes of a α-rhamnosidase and a β-glycosidase. In an advantageous embodiment, in the methods according to the present disclosure such complexes are not used for the production of the natural sweetener composition.

After subjecting the steviol glycoside composition comprising at least the steviol glycoside Dulcoside A and/or Dulcoside B to said enzyme with rhamnosyl hydrolase activity, the resulting steviol glycoside composition is an improved sweetener having a better taste, in particular the composition is sweeter and less bitter than the initial and non enzyme-treated steviol glycoside composition.

In an advantageous embodiment, the methods according to the present disclosure comprises the following steps of:
a) providing a steviol glycoside composition
b) dissolving said steviol glycoside composition
c) providing an enzyme with rhamnosyl hydrolase activity to obtain a reaction mixture
d) incubating the reaction mixture for the de-rhamnosylation of Dulcoside A and/or Dulcoside B
e) separation of the enzyme from the product, and
f) optionally additional down-stream processing of the reaction mixture.

For example, the steviol glycoside composition might be directly produced from leaves and/or other plant materials of *Stevia rebaudisiana* (fresh or dried). Therefore, extracts in aqueous or organic phase are produced by simple infusion or by the usage of extractors (e.g. Soxhlet extractor).

In an advantageous embodiment, the enzyme or the mixture of enzymes may be mixed with the substrate (initial steviol glycoside composition) for the trimming reaction. This can be done in batch mode. Alternatively, fed-batch or continuous mode can facilitate the reaction by avoiding substrate limitations as well as inhibitions caused by substrate, product and/or by-products. Therefore, the application of a membrane reactor can be beneficial. The figure 5 shows a possible setup for a membrane reactor system to enhance productivity by continuous separation of product and/or by-products. Additionally, the membrane holds back the enzymes for reuse within the process. This can improve productivity and reduce the effort of the following purification and downstream process.

The enzyme or mixture of enzymes can be separated (e.g. ultrafiltration) from the reaction solution and be reused. Alternatively, the enzymes or the mixture of enzymes can be immobilised (e.g. on carriers or membranes). In the end by-products like Rhamnose and unwanted steviol glycosides can be separated from the reaction mixture by absorption, adsorption, crystallisation, extraction and membrane processes.

In some embodiments, the non-enzyme treated steviol glycoside composition (initial steviol glycoside composition) is delivered in solid form like powder and must be dissolved in aqueous or organic phase and the enzymatic reaction is executed in an aqueous or organic phase, depending on the nature of the buffer.

In some advantageous embodiments, said enzyme with rhamnosyl hydrolase activity is provided to the dissolved initial steviol glycoside composition to obtain a reaction mixture. The initial steviol glycoside composition may be dissolved in water or organic phase to obtain a solution with 0.5-50%, preferably 5- 30%, more preferably 8-10% (wt/vol) concentration

The pH of the solution is adjusted to pH 3.0-11.5, preferably pH 4.0-10.0, more preferably 5.5 to 8.0, which may be dependend on the pH-stability and/or other properties of the used enzyme or enzyme mixture. The temperature is maintained at a temperature preferably between 20-85 °C, more preferably between 40-80 °C, more preferably between 60-75 °C, which may be depended on the thermo-stable properties of the used enzyme.

Then, an enzyme with rhamnosyl hydrolase activity is added to the solution, obtaining a reaction mixture. The reaction mixture including said enzyme or a mixture of enzymes comprising said enzyme are incubated between 1h and 48h, preferably between 5h and 24h, more preferably between 6 and 12 hour, which may be depended on the amount and activity of the added enzyme. In particular the reaction mixture is incubated until Dulcoside A and/or Dulcoside B are completely or partialially de-rhamnosylated.

In some advantageous embodiments, the conversion rate of Dulcoside A an/or Dulcoside B is preferably > 65%, more preferably > 90%, more preferably > 95 %.

Upon completion, the reaction mixture may be optionally further processed (down-stream processing), in particular for further bitterness reduction and/or separation of by-products. As mentioned above, Rhamnose and/or Rubusoside may be separated from the natural sweetener composition by absorption, adsorption, crystallization, extraction, and membrane or chromatography process.

In advantageous embodiments, after the enzyme treatment, the resulting reaction mixture (natural sweetener composition) Dulcoside A is contained in the reaction mixture at least at a concentration below 49 µM, which is the lower threshold concentration for bitterness. Dulcoside B is contained in the reaction mixture at least at a concentration below 49 µM, which is the lower threshold concentration for bitterness.

In some further advantageous embodiments, the concentration of Rubusoside in the in the reaction mixture is lower than 61 µM but higher than > 27.3 µM (between threshold concentrations bitterness and sweetness).

Therefore, the present disclosure pertains also to a natural sweetener composition derived from a *Stevia rebaudiana* plant extract containing the steviol glycoside Dulcoside A at a concentration of less than 2 % (wt/vol), preferably less than 0.5 % (wt/vol), more preferably less than 0.1 % (wt/vol) and the steviol glycoside Dulcoside B at a concentration of less than 2 % (wt/vol), preferably less than 0.5 % (wt/vol), more preferably less than 0.1 % (wt/vol).

In another aspect, the present disclosure pertains to natural sweetener compositions obtainable by a method according to the present disclosure, wherein said composition contains Dulcoside A at a concentration of less than 2 % (wt/vol), preferably less than 0.5 % (wt/vol), more preferably less than 0.1 % (wt/vol) and Dulcoside B at a concentration of less than 2 % (wt/vol), preferably less than 0.5 % (wt/vol), more preferably less than 0.1 % (wt/vol).

In advantageous embodiments, the natural sweetener compositions are obtainable by a method according to the present disclosure, in particular by subjecting a steviol glycoside composition comprising at least the steviol glycoside Dulcoside A and/or Dulcoside B to an enzyme with rhamnosyl hydrolase activity.

In a further advantageous embodiment, the methods according to the present disclosure comprise the following steps:
- providing an enzyme or a mixture of enzymes with rhamnosyl hydrolase activity
- providing the steviol glycoside composition
- dissolving the steviol glycoside composition
- incubating the reaction mixture (steviol glycoside mixture and enzyme or mixture of enzymes) to facilitate complete or partial de-rhamnosylation of Dulcoside A and/or Dulcoside B
- separation of the enzyme from the product, and
- optionally additional down-stream processing of the reaction mixture for further bitterness reduction and/or separation of by-products.

In one embodiment of the present disclosure, Stevia extract commercialized by impag (Switzerland) containing Stevioside (45%), Rebaudioside A (40%), Dulcoside A (5%), Rebaudioside C (2,5%)and other glycosides amounting to total steviol glycosides' content of at least 95 %, was used as a starting material. Alternatively stevia extracts with different ratio of steviol glycosides may be used as starting materials.

The starting material was subjected to the enzymatic de-rhamnosylation by action of Rhamnosidase. The de-rhamnosylation of Dulcoside A results in Rubusoside. The de-rhamnosylation of Dulcoside B yields 13-O-β-Laminaribiosyl-19-O-β-D-Glucosyl-Steviol (a stereo isomer from Stevioside) that can be synthesised in *S. rebaudiana* by glycosylation of Rubusoside and is a precursor in biosynthesis of Rebaudioside A. The de-rhamnosylation yields α-L-rhamnose which is also a valuable product.

Preferably, the molar yield of Dulcoside A and/or Dulcoside B conversion to de-rhamnosyl steviol glycosides is more than 65 %, preferably more than 90 %, more preferably more than 95 %.

In another advantageous embodiments, natural sweetener compositions of the present disclosure are obtainable by the above mentioned methods, wherein said composition contains Dulcoside A at a concentration of less than 2 % (wt/vol), preferably less than 0.5 % (wt/vol), more preferably less than 0.1 % (wt/vol) and Dulcoside B at a concentration of less than 2 % (wt/vol), preferably less than 0.5 % (wt/vol), more preferably less than 0.1 % (wt/vol).

The natural sweetener compositions according to the present disclosure and/or produced by a method according to the present disclosure can be used as sweeteners in various food and beverage products. Non-limiting examples of food and beverage products include carbonated soft drinks, ready to drink beverages, energy drinks, isotonic drinks, low-calorie drinks, zero-calorie drinks, sports drinks, teas, fruit and vegetable juices, juice drinks, dairy drinks, yoghurt drinks, alcohol beverages, powdered beverages, bakery products, cookies, biscuits, baking mixes, cereals, confectioneries, candies, toffees, chewing gum, dairy products, flavoured milk, yoghurts, flavoured yoghurts, cultured milk, soy sauce and other soy base products, salad dressings, mayonnaise, vinegar, frozen-desserts, meat products, fish-meat products, bottled and canned foods, table top sweeteners, fruits and vegetables.

In figure 1 the reaction catalyzed by Rhamnosidase is shown. The two rhamno steviol glycosides Dulcoside A and Dulcoside B (respectively Rebaudioside C) are known to cause more bitter taste than other steviol glycosides. Their terminal rhamnosyl residue can be trimmed using a Rhamnosidase. The selected Rhamnosidase is capable to hydrolyse the linkage between the glycosyl and the rhamnosyl residue. The de-rhamnosylation of Dulcoside A yields in Rubusoside, which is known to be less bitter and sweeter than Dulcoside A. The conversion of Dulcoside B produces De-rhamno-Dulcoside B, which is a stereoisomer of Stevioside. In Stevioside the glycosidic bond between the two glycosyl residues is β-(2-1) in the isomer it is a β-(3-1) glycosidic bond.

In figure 2 the chromatographical analysis of a mixture of five different HPLC analytes is shown. Each analyte has a concentration of 0.2 mg/ml. The sample was analysed with Eurospher 100-5 NH2, 150 x 3 mm column (Knauer, Germany) and detected on 210 nm. The mobile phase consisted of 80% MeCN and 20% H2O. All peaks showed comparable peak areas. With rising length of the sugar residue chain the symmetry of the peaks changed. While the signal of Rubusoside was symmetric and showed a short full width at half maximum, the signal of Rebaudioside A showed strong fronting with a long full width at half maximum.

In figure 3 the conversion of Dulcoside A to Rubusoside in a commercial mixture of steviol glycosides using a fungal α-rhamnosidase from *Aspergillus sp*.is shown as an example. On a thermal shaker 100 U of enzyme were incubated with 0.2 mg mixture of steviol glycosides for 12 h in 200 µl of 50 mM phosphate buffer at pH 6.0 and 60°C. The enzyme was separated via membrane filtration in spin columns. A second mixture without enzyme solution was incubated at the same conditions for background measurement. The comparison of both chromatograms showed a significant reduction of the signal for Dulcoside A at 6.45 min and a new signal for Rubusoside at 4.35 min after 12 h of incubation. The signals of Stevioside and Rebaudioside A did not change at all. The specific enzyme used in this reaction had no glycosyl hydrolase activity against the steviol glycosides in the mixture.

### EXAMPLES

In the following examples, materials and methods of the present disclosure are provided. It should be understood that these examples are for illustrative purpose only and are not to be construed as limiting this disclosure in any manner. All publications, patents, and patent applications cited herein are hereby incorporated by reference in their entirety for all purposes.

### 1. Preparation of enzyme with rhamnosyl hydrolase activity

Via recombinant cloning techniques the DNA coding for different rhamnosidases orginated from bacteria or fungi has been cloned into the genome of the yeast *Pichia pastoris.* The enzymes were secreted in to the culture medium using the α-mating factor pre/pro-sequence.

After cultivation in a lab-scale bioreactor (5 L) the supernatant was harvested via sterile filtration using ceramic membranes with a cut-off of 0.25 nm. The supernatant had an activity of 9.5 U/mL with 50 U/mg of total protein. The enzyme in the supernatant was concentrated in another ceramic membrane module with a cut-off of 20 kDa. Via diafiltration with 50 mM phosphate buffer (pH 6) the enzyme was washed. After concentrating and washing the activity of the enzyme solution was about 1000 U/mL with 250 U/mg total protein. Figure 4 shows the schematic setup of the protein production process. The enzymes were assayed on glycosyl hydrolase and rhamnosyl hydrolase activity using p-nitrophenyl beta-D-glucoside and p-nitrophenyl alpha-L-rhamnoside. No glycosyl hydrolase activities were measured (Romero et al, 1985, Anal. Biochem. 2, P. 566-571.).

### 2. Enzymatically conversion of Dulcosides

### a) Experiments with pure Dulcosides

In a first approach aqueous solutions of pure **Dulcosides** were assayed with different enzymes (rhamnosyl hydrolases) to select an enzyme capable to transform **Dulcosides** to de-rhamno steviol glycosides. These enzymes were chosen from the group of: *Aspergillus* sp., *Bacillus* sp., *Clostridium* sp., *Lactobacillus* sp., *Thermobacteria* sp., *Penicillium* sp. and others. On a thermal shaker 100 U of enzyme were incubated with 0.1 mg pure Substrate for 12 h in 200 µl 50 mM phosphate buffer at pH 6.0 and 60°C. The enzymes were separated via membrane filtration in spin columns or the enzymes were precipitated by the addition of acetonitrile (MeCN). Steviol glycosides were analysed via High-performance liquid chromatography (HPLC) using Eurospher 100-5 NH2, 150 x 3 mm column (Knauer, Germany). The mobile phase consisted of 80% MeCN and 20% H2O. The figure 2 shows a chromatogram of a standard mixture of major steviol glycosides found in stevia extracts. Enzymes capable to convert Dulcosides were selected.

Fig. 1 shows the conversion of rhamno steviol glycosides by α-Rhamnosidase. The trimming of Rhamnose from Dulcoside A yields in Rhamnose and Rubusoside. The conversion of Dulcoside B yields Rhamnose and de-rhamno-Dulcoside B, which is a stereoisomer of Stevioside. This stereoisomer can also be produced in the plant *Stevia rebaudisiana.*

### b) Experiments with commercial steviol glycoside mixtures

Selected enzymes converting **Dulcosides** were tested in commercial mixtures (impag, Switzerland; Daepyung Co., Ltd., Korea; Xian Greenherb Biotech Co., Ltd., China for the specific derhamnosylation of **Dulcosides** in the presence of other steviol glycosides like Stevioside and Rhamnoside A. On a thermal shaker 100 U of enzyme were incubated with 0.1 mg mixtures of steviol glycosides for 12 h in 200 µl 50 mM phosphate buffer at pH 6.0 and 60°C. A second mixture without enzyme solution was incubated at the same conditions for background measurement. The enzymes were separated via membrane filtration in spin columns or the enzymes were precipitated by the addition of acetonitrile (MeCN). The steviol glycosides were analyzed as described above. In figure 3 the specific conversion of Dulcoside A to Rubusoside in a commercial mixture of steviol glycosides using a fungal α-rhamnosidase from *Aspergillus sp.* is shown as an example. The comparison of both chromatograms (incubation with and without enzyme) showed a significant reduction of the signal for Dulcoside A at 6.45 min and a new signal for Rubusoside at 4.35 min after 12 h of incubation. The signals of Stevioside and Rebaudioside A did not change at all. The specific enzyme used in this reaction had no glycosyl hydrolase activity against the steviol glycosides in the mixture. Moreover, in this special case the utilized enzyme was so specific that no de-rhamnosylation of Dulcoside B was observed.

Enzymes, which were capable to trim Rhamnose specifically from **Dulcosides** in steviol glycoside mixtures, were selected.

### c) Experiments with fresh aqueous extracts from Stevia rebaudisiana leaves

In the third approach steviol glycosides were extracted from leaves of *Stevia rebaudisiana* by simple infusion or using a Soxhlet extractor. On a thermal shaker 100 U of enzyme were incubated with 0.1 mg extracts for 12 h in 200 µl 50 mM phosphate buffer at pH 6.0 and 60°C. The enzymes were separated via membrane filtration in spin columns or the enzymes were precipitated by the addition of acetonitrile (MeCN). The steviol glycosides were analyzed as described above. Enzymes capable to trim Rhamnose specifically from **Dulcosides** in stevia extracts were selected and characterized on their biophysical and catalytic properties.

## Claims

1. A method for producing a natural sweetener composition comprising a plurality of sweetener by subjecting a steviol glycoside composition comprising at least the steviol glycoside Dulcoside A and/or Dulcoside B to an enzyme with rhamnosyl hydrolase activity.

2. The method according to claim 1, wherein the method comprises the following steps of:
a) providing the steviol glycoside composition
b) dissolving the steviol glycoside composition
c) providing an enzyme with rhamnosyl hydrolase activity to obtain a reaction mixture
d) incubating the reaction mixture for the de-rhamnosylation of Dulcoside A and/or Dulcoside B
e) separation of the enzyme from the product, and
f) optionally additional down-stream processing of the reaction mixture for further bitterness reduction and/or separation of by-products.

3. The method according to any one of claims 1 to 2, wherein the steviol glycoside composition is derived from *Stevia rebaudiana* Bertoni leaves.

4. The method according to any one of claims 1 to 3, wherein the steviol glycoside composition is a *Stevia rebaudiana* plant extract.

5. The method according to any one of claims 1 to 4, wherein the steviol glycoside composition is a mixture of steviol glycosides comprising at least Stevioside, Rebaudiosides A, Dulcoside B (Rebaudiosides C) and Dulcoside A.

6. The method according to any one of claims 1 to 5, wherein the molar yield of Dulcoside A and/or Dulcoside B conversion to de-rhamnosyl steviol glycosides is more than 65 %, preferably more than 90 %, more preferably more than 95 %.

7. The method according to any one of claims 1 to 6, wherein step (b) is executed in aqueous or organic phase.

8. The method according to any one of claims 1 to 7, wherein said enzyme with rhamnosyl hydrolase activity is provided in free or immobilized form.

9. The method according to any one of claims 1 to 8, wherein said enzyme with rhamnosyl hydrolase activity, has no glycosyl hydrolase activity and/or has no specific glycosyl hydrolase activity against steviol glycosides.

10. The method according to claim 9, wherein no further enzyme with glycosyl hydrolase activity is added to the reaction mixture, in particular the further enzyme having glycosyl hydrolase activity is selected from the group consisting of ß-glucosidase, hesperidinase, naringinase, pectinase, cellulase, in free or immobilized forms.

11. The method according to any one of claims 1 to 10, wherein the enzyme with rhamnosyl hydrolase activity is derived from bacteria, in particular from bacteria of the order Thermales or said enzyme is derived from fungi, in particular from a fungi selected from the group consisting of Basidiomycota, Ascomycota and Saccharomycotina.

12. The method according to any one of claims 1 to 10, wherein the enzyme with rhamnosyl hydrolase activity is an artificial technical enzyme.

13. The method according to any one of claims 1 to 12, wherein step (d) is executed in a stirred tank reactor in batch mode, in fed-batch mode or continuous mode.

14. The method according to any one of claims 1 to 13, wherein step (f) Rhamnose and/or Rubusoside is separated from the natural sweetener composition by absorption, adsorption, crystallization, extraction, membrane or chromatography process.

15. A natural sweetener composition obtainable by a method according to any one of claims 1 to 14, wherein said composition contains Dulcoside A at a concentration of less than 2 % (wt/vol), preferably less than 0.5 % (wt/vol), more preferably less than 0.1 % (wt/vol) and Dulcoside B at a concentration of less than 2 % (wt/vol), preferably less than 0.5 % (wt/vol), more preferably less than 0.1 % (wt/vol).
